# EUROPÄISCHE PATENTSCHRIFT

(11) **EP 0 869 127 B1**
(45) Veröffentlichungstag und Bekanntmachung des Hinweises auf die Patenterteilung: **10.10.2001**
(21) Anmeldenummer: 98110227.0
(22) Anmeldetag: 10.11.1994
(51) Int. Cl.: C07D 487/04

(54) **Bicyclische Amidine und ihre Verwendung als Katalysator**
Bicyclic amidines and their use as catalysts
Amidines bicycliques et leur utilisation comme catalyseurs

(30) Priorität: 11.11.1993 CH 338593
(43) Veröffentlichungstag der Anmeldung: 07.10.1998
(62) Teilanmeldung aus: 94117768.5
(73) Patentinhaber: Lonza AG, 3930 Visp (CH)
(72) Erfinder: Werbitzky, Oleg, Dr., 3930 Visp (Kanton Wallis) (CH); Daum, Ulrich, Dr., 4114 Hofstetten (Kanton Solothurn) (CH); Bregy, Rachel, 3942 Raron (Kanton Wallis) (CH)
(74) Vertreter: Winter, Brandl, Fürniss, Hübner, Röss, Kaiser, Polte Partnerschaft

(56) Entgegenhaltungen:
- EP-A- 0 199 483
- GB-A- 1 182 014
- MOEHRLE,H;OTTERSBACH,D; STEIGEL,A: "Ring-chain isomerism of fused alpha-amino nitrones" PHARMAZIE, Bd. 47, Nr. 6, 1992, Seiten 403-409, XP002073800

## Beschreibung

Die vorliegende Erfindung betrifft neue bicyclische Amidine mit funktionellen Gruppen, insbesondere Amino-, Hydroxy und / oder Mercaptogruppen, deren Verwendung als Katalysatoren für die Herstellung von Polyurethanen, und die Herstellung der neuen Verbindungen durch Umsetzung von Lactonen mit primären Aminen.

Bicyclische Amidine sind starke organische Basen, die wegen ihrer hohen Basizität bei geringer Nucleophilie und ihrer guten Löslichkeit in fast allen Lösungsmitteln zahlreiche Anwendungen gefunden haben. Besonders bekannt sind die üblicherweise mit den Abkürzungen DBN und DBU bezeichneten Verbindungen 1,5-Diazabicyclo[4.3.0]non-5-en (2,3,4,6,7,8-Hexahydropyrrolo[1,2-a]pyrimidin) und 1,8-Diazabicyclo[5.4.0]undec-7-en (2,3,4,6,7,8,9,10-Octahydropyrimido[1,2-a]azepin).

Eine Übersicht über Verwendungen dieser Verbindungen in chemischen Synthesen findet sich z. B. in "Synthetica Merck", Band II, E. Merck, Darmstadt, 1974, S. 118-119, 124.

Ein bekanntes Herstellungsverfahren für bicyclische Amidine geht von N-(ω-Aminoalkyl)lactamen aus, die beim Erhitzen mit sauren Katalysatoren unter Wasserabspaltung zu den Amidinen cyclisieren (DE-PS 15 45 855).
Die N-(ω-Aminoalkyl)lactame werden beispielsweise aus den entsprechenden Cyanoverbindungen durch Hydrierung erhalten, so insbesondere das N-(γ-Aminopropyl)pyrrolidon aus dem N-(ß-Cyanethyl)pyrrolidon (siehe z. B. W. Reppe et al., Justus Liebigs Ann. Chem. 1955,' 596, S. 211).
Es ist auch möglich, N-(ω-Aminoalkyl)lactame aus den entsprechenden Lactonen und α,ω-Diaminoalkanen herzustellen (DE-PS 730 182).
Die bekannten Verfahren zur Herstellung bicyclischer Amidine haben den Nachteil, dass sie mindestens zwei Synthesestufen mit Aufarbeitung der Zwischenprodukte umfassen.

Es ist bekannt, dass sich bicyclische Amidine gut als Katalysatoren für die Herstellung von Polyurethanen eignen (FR-PS 1 542 058).
Ein erheblicher Nachteil liegt jedoch darin, dass sie im gebildeten Polyurethan nicht fest gebunden werden und daher mit der Zeit aus diesem herausdiffundieren oder herausgelöst werden. Dies kann zu Beeinträchtigungen von Materialien in der Umgebung oder sogar zur Schädigung oder Belästigung von Personen, welche mit dem Polyurethan in Kontakt kommen, führen. In jedem Fall bedeutet es eine unnötige Belastung der Umwelt.

Aufgabe der vorliegenden Erfindung war daher, neue Verbindungen aus dieser Verbindungsklasse bereitzustellen, welche bei der Verwendung als Katalysator zur Herstellung von Polyurethanen so fest an das Polymer gebunden werden, dass sie keine nennenswerte Migrationstendenz mehr aufweisen.

Erfindungsgemäss wird die Aufgabe durch die neuen Verbindungen nach Patentanspruch 1 gelöst.

Es wurde gefunden, dass es möglich ist, bicyclische Amidine der allgemeinen Formel 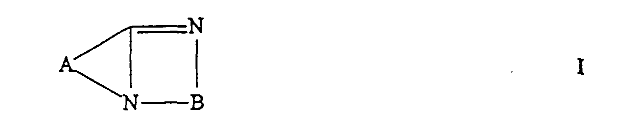 in einem Eintopfverfahren ohne Isolierung oder Reinigung von Zwischenstufen aus den entsprechenden Lactonen der allgemeinen Formel 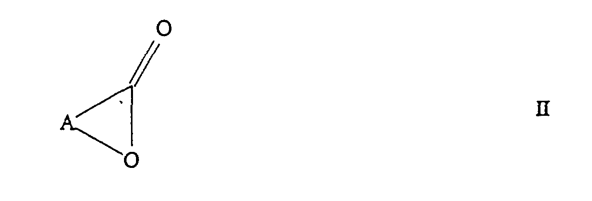 und Aminen der allgemeinen Formel

H₂N―B―NH₂ III

herzustellen.

Die Gruppe A im Lacton (II) und dem Amidin (I) steht jeweils für eine 3-, 4- oder 5-gliedrige Kohlenstoffkette der Formel -CR¹R²-CR³R⁴-CR⁵R⁶-,
-CR¹R²-CR³R⁴-CR⁵R⁶-CR⁷R⁸- oder -CR¹R²-CR³R⁴-CR⁵R⁶-CR⁷R⁸-CR⁹R¹⁰-, wobei R¹ und R² jeweils an das dem Heteroatom benachbarte Kohlenstoffatom gebunden sind.
Die Gruppe B im Amin (III) und dem Amidin (I) steht jeweils für eine 2-, 3- oder 4-gliedrige Kohlenstoffkette der Formel -CR¹¹R¹²-CR¹³R¹⁴-, -CR¹¹R¹²-CR¹⁵R¹⁶-CR¹³R¹⁴- oder -CR¹¹R¹²-CR¹⁵R¹⁶-CR¹⁷R¹⁸-CR¹³R¹⁴-.

Damit umfasst die allgemeine Formel I bicyclische Amidine mit 5-, 6- oder 7-gliedrigen Ringen, wobei die beiden Ringe gleiche oder verschiedene Gliederzahl aufweisen können:
Entsprechend umfasst die allgemeine Formel II Lactone mit 5 bis 7 Ringgliedern, also γ-, δund ε-Lactone.
Die Substituenten R¹, R² und R¹ bis R¹⁴ der Kohlenstoffketten A und B sind jeweils unabhängig voneinander Wasserstoff, C₁-C₄-Alkyl oder Aryl oder C₁-C₄-Alkylgruppen, welche ihrerseits mit Hydroxy, Amino, C₁-C₄-Alkylamino oder Mercapto substituiert sind. Die Substituenten R³ bis R¹⁰ und R¹⁵ bis R¹⁸ können ausser den für R¹, R² und R¹¹ bis R¹⁴ genannten Gruppen noch Hydroxy-, Amino-, C₁-C₄-Alkylamino- oder Mercaptogruppen sein.
Unter C₁-C₄-Alkyl sind hier alle primären, sekundären und tertiären unverzweigten oder verzweigten Alkylgruppen mit bis zu 4 Kohlenstoffatomen zu verstehen, also Methyl, Ethyl, Propyl, Butyl, Isopropyl, Isobutyl, sec-Butyl und tert-Butyl. Unter Aryl ist insbesondere Phenyl oder alkylsubstituiertes Phenyl zu verstehen, wie beispielsweise o-, m- oder p-Tolyl oder die verschiedenen isomeren Xylylgruppen. Als Lactone eignen sich beispielsweise γ-Butyrolacton, γ- und δ-Valerolacton, ε-Caprolacton oder substituierte Lactone wie Pantolacton (2-Hydroxy-3,3-dimethyl-y-butyro-lacton).
Funktionelle Gruppen als Substituenten, also Hydroxy, Amino, Alkylamino oder Mercapto, befinden sich vorzugsweise an der Gruppe B der Aminkomponente (III).
Als Amine (III) kommen daher nicht nur primäre Diamine, sondern auch Verbindungen mit zusätzlichen primären oder sekundären Aminogruppen in Frage. Geeignete Amine sind beispielsweise 1,2-Diaminoethan, 1,2-Diaminopropan, 1,3-Diaminopropan, 1,2,3-Triaminopropan, 1,1,1-Tris(aminomethyl)ethan oder Tetrakis(aminomethyl)methan. Werden Amine mit nicht-äquivalenten primären Aminogruppen eingesetzt, wie beispielsweise 1,2-Diaminopropan oder 1,2,3-Triaminopropan, so können unter Umständen Produktgemische entstehen.

Bei der Umsetzung des Lactons (II) mit dem Amin (III) wird zweckmässig ein molares Verhältnis von wenigstens 1 mol Amin auf 1 mol Lacton eingehalten. Vorzugsweise werden 2 bis 20 mol Amin auf 1 mol Lacton eingesetzt. Der Überschuss an Amin kann bei der Aufarbeitung des Reaktionsgemischs zurückgewonnen werden.

Die Reaktion wird zweckmässig bei einer Temperatur von mindestens 150°C durchgeführt. Vorzugsweise liegt die Reaktionstemperatur zwischen 200 und 300°C. Ein inertes Lösungsmittel wie beispielsweise Toluol oder Xylol kann zugesetzt werden, ist aber nicht erforderlich. Vorzugsweise wird die Reaktion ohne Lösungsmittel durchgeführt. Zur Erreichung der Reaktionstemperatur ist es im allgemeinen erforderlich, das Reaktionsgemisch unter erhöhtem Druck zu halten, weil die Siedepunkte vieler Edukte bei Normaldruck niedriger als die Reaktionstemperatur liegen. Hierzu können gewöhnliche Autoklaven eingesetzt werden. Zur Beschleunigung der Reaktion wird vorzugsweise ein saurer Katalysator zugesetzt. Hierfür eignen sich Brønsted-Säuren wie beispielsweise Salzsäure, Schwefelsäure, Phosphorsäure oder Ammoniumchlorid oder auch saure Aluminiumsilikate oder saure Metalloxide wie beispielsweise Zinn(IV)oxid oder Antimon(III)oxid.

Das Reaktionsgemisch wird ohne Isolierung eines Zwischenprodukts direkt destilliert. Aus praktischen Gründen wird es hierzu typischerweise aus dem Autoklaven in eine Destillationsapparatur übergeführt. Falls eine entsprechend ausgerüstete Apparatur, die sich sowohl für Überdruck als auch für Vakuum eignet, zur Verfügung steht, können selbstverständlich die Umsetzung des Lactons mit dem Amin und die Destillation in ein und derselben Apparatur durchgeführt werden. Bei der Destillation geht zunächst das beim Ringschluss entstandene Wasser und das überschüssige Amin über, danach das Amidin. Je nach Siedepunkt des Produkts wird die Destillation bei entsprechend vermindertem Druck durchgeführt.

Nach dem beschriebenen Verfahren können nicht nur die in der Einleitung erwähnten bekannten bicyclischen Amidine wie beispielsweise DBN und DBU hergestellt werden, sondern insbesondere auch neue Verbindungen aus dieser Stoffklasse mit bisher unerreichten Eigenschaften.

Es wurde gefunden, dass solche bicyclischen Amidine (1), in denen wenigstens einer der Substituenten R¹ bis R¹⁸ eine primäre oder sekundäre Aminogruppe, eine Hydroxygruppe und / oder eine Mercaptogruppe ist bzw. trägt, als Katalysator für die Herstellung von Polyurethanen verwendet werden können und so fest im Polymer gebunden werden, dass weder im Gebrauch noch bei den üblichen Extraktionstests eine Migration feststellbar ist. Vermutlich reagieren diese zusätzlichen funktionellen Gruppen bei der Herstellung des Polyurethans mit den Isocyanatgruppen der Isocyanatkomponente des Polyurethans unter Ausbildung kovalenter Bindungen.
Die zusätzlichen funktionellen Gruppen können selbstverständlich auch zu anderen organischen Reaktionen mit polymeren oder nichtpolymeren Isocyanaten, Epoxiden, Carbonsäuren, Carbonsäurederivaten oder anderen Verbindungen herangezogen werden.
Vorzugsweise sind die zusätzlichen funktionellen Gruppen Amino- oder Aminoalkylgruppen wie beispielsweise Aminomethylgruppen. Sie befinden sich vorzugsweise an der in der allgemeinen Formel I mit "B" bezeichneten Kette, also in den Positionen der Substituenten R¹¹ bis R¹⁸.

Besonders bevorzugt sind die Verbindungen
3-Amino-2,3,4,6,7,8-hexahydropyrrolo[1,2-a]pyrimidin 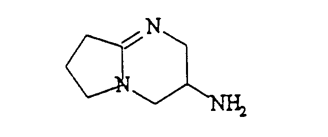
3-(Aminomethyl)-2,5,6,7-tetrahydro-3*H*-pyrrolo[1,2-a]imidazol 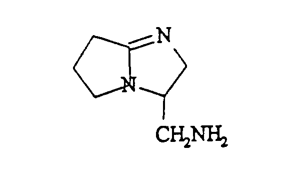 sowohl einzeln als auch als Gemisch
und 3-(Aminomethyl)-3-methyl-2,3,4,6,7,8-hexahydropyrrolo[1,2-a]pyrimidin

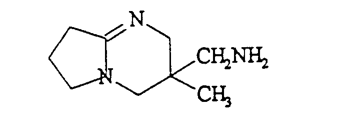

Die nachfolgenden Beispiele verdeutlichen die Durchführung des Verfahrens sowie die Eigenschaften und Verwendungen der erfindungsgemässen Verbindungen.

### Beispiel 1

### Herstellung von 3-Amino-2,3,4,6,7,8-hexahydropyrrolo[1,2-alpyrimidin und 3-(Aminomethyl)2,5,6,7-tetrahydro-3H-pyrrolo[1,2-a]imidazol

Analog zu Beispiel 1 wurden 21,52 g (0,25 mol) γ-Butyrolacton, 1,34 g (25 mmol) Ammoniumchlorid und 83,43 g (0,94 mol) 1,2,3-Triaminopropan umgesetzt. Der nach Abdestillieren des überschüssigen Amins und des Wassers verbleibende Rückstand wurde bei 170 - 200°C / 0,1 mbar (Badtemperatur) destilliert.
Ausbeute: 22,2 g (64%) gelbliches Öl
Kp: 95 - 97°C / 2 mbar
Das Produkt bestand nach GC zu ca. 90% aus dem Isomeren mit Pyrrolo[1,2-a]pyrimidin-Gerüst und zu ca. 10% aus dem Isomeren mit Pyrrolo[1,2-a]imidazol-Gerüst.

| 3-Amino-2,3,4,6,7,8-hexahydropyrrolo[1,2-a]pyrimidin | |
|---|---|
| ¹H-NMR (CDCl₃, 400 MHz) δ | 1,96 (quint, 2H, H-7); |
| | 2,45 (t, 2H, H-8); |
| | 2,7 - 2,8 (m, 1H, H-4ₐ); |
| | 3,05 (t, 1H, H-2ₐ); |
| | 3,1-3,2 (m, 1H, H-3); |
| | 3,3 (t, 2H, H-6); |
| | 3,35 (t, 1H, H-4_{b}); |
| | 3,45-3,5 (m, 1H, H-2_{b}). |
| | |
| | |
| ¹³C-NMR (100 MHz) δ | 160,5 (s, C-8a); |
| | 52,88 (t, C-2); |
| | 51,4 (t, C-6); |
| | 50,9 (t, C-4); |
| | 43,0 (t, C-3); |
| | 30,9 (t, C-8); |
| | 20,0 (t, C-7). |

| 3-(Aminomethyl)-2,5,6,7-tetrahydro-3*H*-pyrrolo[1,2-a]imidazol | |
|---|---|
| ¹H-NMR (CDCl₃, 400 MHz) δ | 2,28-2,32 (m, 2H, H-6); |
| | 2,32 - 2,35 (m, 2H, H-7); |
| | 2,75 (m, 1H, CHₐNH₂); |
| | 2,85 (m, 2H, H-5); |
| | 3,3 (m, 1H, CH_{b}NH₂); |
| | 3,4 (m, 1H, H-3); |
| | 3,8 (dd, 1H, H-2ₐ); |
| | 4,15 (dd, 1H, H-2_{b}). |
| | |
| ¹³C-NMR (CDCl₃, 100 MHz) δ | 175,8 (s, C-7a); |
| | 64,5 (t, C-2); |
| | 64,3 (d, C-3); |
| | 45,8 (t, CH₂NH₂); |
| | 44,3 (t, C-5); |
| | 25,4 (t, C-7); |
| | 22,6 (t, C-6). |

### Beispiel 2

### Herstellung von 3-(Aminomethyl)-3-methyl-2,3,4,6,7,8-hexahydropyrrolo[1,2-a]pyrimidin

Ein Gemisch von 21,03 g (0,24 mol) γ-Butyrolacton und 1,31 g (24 mmol) Ammoniumchlorid in 114,5 g (0,98 mol) 1,1,1-Tris(aminomethyl)ethan wurde in einem Autoklaven auf 250°C erhitzt. Nach 1,5 h wurde das Gemisch abgekühlt und das überschüssige Amin und das entstandene Wasser abdestilliert. Der Rückstand wurde bei 200 - 240°C / 18 mbar destilliert. Ausbeute: 22,0 g (54%) gelbliches Öl
Kp: 117 - 120°C / 1 mbar

| | |
|---|---|
| ¹H-NMR (CDCl₃, 400 MHz) δ | 0,91 (s, 3H, CH₃); |
| | 1,95 (quint, 2H, H-7); |
| | 2,45 (t, 2H, H-8); |
| | 2,53 (dd, 2H, CH₂NH₂); |
| | 2,84 (dd, 1H, H-4ₐ); |
| | 3,0 (m, 2H, H-2ₐ, H-4_{b}); |
| | 3,13 (dd, 1H, H-2_{b}); |
| | 3,28 (dt, 2H, H-6). |
| | |
| ¹³C-NMR (CDCl₃, 100 MHz) δ | 160,1 (s, C-8a); |
| | 53,5 (t, C-2); |
| | 51,5 (t, C-6); |
| | 51,3 (t, C-4) |
| | 48,5 (t, CH₂NH₂); |
| | 32,0 (s, C-3); |
| | 30,9 (t, C-8); |
| | 20,9 (q, CH₃); |
| | 19,9 (t, C-7). |

### Beispiele 3-5

### Herstellung von Polyurethanen mit bicyclischen Amidinen als Katalysator

Allgemeine Vorschrift:
Bei Raumtemperatur wurden 50 g eines aromatischen Polyisocyanats aufBasis von Diphenylmethandiisocyanat (ca 32% NCO) unter Rühren mit einem Glasstab zu einer Lösung von 0,5 g des Amidins in 55 g eines trifunktionellen hydroxylhaltigen verzweigten Polyethers (Desmophen® 550 U, ca. 11,5% OH) gegeben. Aus dem Gemisch wurde jeweils eine Folie von ca. 200 µm Dicke gegossen. Nach dem Aushärten wurde die Migrationsfähigkeit des Amidins durch Extraktionsversuch bestimmt. Dazu wurden jeweils 10 g der so erhaltenen Folien in kleine Stücke geschnitten und in 40 ml Methanol bei Raumtemperatur aufbewahrt. Nach verschiedenen Zeiten wurde in Proben des Methanols mittels GC der Amidingehalt bestimmt.

### Beispiel 3 (Vergleichsbeispiel)

Als Amidin wurde 2,3,4,6,7,8-Hexahydropyrrolo[1,2-a]pyrimidin (DBN) eingesetzt. Die Gelzeit betrug 3 min 15 s.

| | | |
|---|---|---|
| Amidingehalt im Extraktionsversuch | nach 1 h | ca. 0,012 % |
| | nach 1 Tag | ca. 0,06 % |
| | nach 14 Tagen | ca. 0,1% |

Nach 14 Tagen war somit der grösste Teil des insgesamt vorhandenen Amidins extrahiert.

### Beispiel 4

Als Amidin wurde ein Gemisch aus ca. 90% 3-Amino-2,3,4,6,7,8-hexahydropyrrolo-[1,2-a]pyrimidin und ca. 10% 3-(Aminomethyl)-2,5,6,7-tetrahydro-3*H*-pyrrolo-[1,2-a]imidazol (hergestellt nach Beispiel 1) eingesetzt. Die Gelzeit betrug 3 min 30 s.

| | | |
|---|---|---|
| Amidingehalt | nach 1 h | <10 ppm |
| | nach 1 Tag | <10 ppm |
| | nach 14 Tagen | <10 ppm |

### Beispiel 5

Als Amidin wurde 3-(Aminomethyl)-3-methyl-2,3,4,6,7,8-hexahydropyrrolo[1,2-a]pyrimidin (hergestellt nach Beispiel 2) eingesetzt. Die Gelzeit betrug 3 min.

| | | |
|---|---|---|
| Amidingehalt | nach 1 h | <10 ppm |
| | nach 1 Tag | <10 ppm |
| | nach 14 Tagen | <10 ppm |

Bei den erfindungsgemässen Amidinen lag die extrahierte Menge selbst nach 14 Tagen noch unter der Nachweisgrenze.

### Beispiele 6-8

Allgemeine Vorschrift:
Bei Raumtemperatur wurden 40 g eines aliphatischen Triisocyanats (ca. 23% NCO) unter Rühren mit einem Glasstab zu einer Lösung von 0,44 g des Amidins und 30 mg Dibutylzinndilaurat in einem Gemisch aus je 24 g eines trifunktionellen hydroxylhaltigen verzweigten Polyethers (Desmophen® 550 U, ca. 11,5 % OH und Desmophen® 1915 U, ca. 1,1 % OH) gegeben. Das Gemisch wurde zu einer Folie von ca. 200 µm Dicke gegossen, mit welcher wie in den Beispielen 3 bis 5 verfahren wurde.

### Beispiel 6 (Vergleichsbeispiel):

Als Amidin wurde 2,3,4,6,7,8-Hexahydropyrrolo[1,2-a]pyrimidin (DBN) eingesetzt. Die Gelzeit betrug 70 s.

| | | |
|---|---|---|
| Amidingehalt im Extraktionsversuch | nach 1 Tag | <10 ppm |
| | nach 14 Tagen | <10 ppm |
| | nach 2 Monaten | 0,03% |

### Beispiel 7

Als Amidin wurde ein Gemisch aus ca. 90% 3-Amino-2,3,4,6,7,8-hexahydropyrrolo-[1,2-a]pyrimidin und ca. 10% 3-(Aminomethyl)-2,5,6,7-tetrahydro-3*H*-pyrrolo-[1,2-a]imidazol (hergestellt nach Beispiel 1) eingesetzt. Die Gelzeit betrug 75 s.

| | | |
|---|---|---|
| Amidingehalt | nach 1 h | <10 ppm |
| | nach 14 Tagen | <10 ppm |
| | nach 2 Monaten | <10 ppm |

### Beispiel 8

Als Amidin wurde 3-(Aminomethyl)-3-methyl-2,3,4,6,7,8-hexahydropyrrolo[1,2-a]pyrimidin (hergestellt nach Beispiel 2) eingesetzt. Die Gelzeit betrug 65 s.

| | | |
|---|---|---|
| Amidingehalt | nach 1 h | <10 ppm |
| | nach 1 Tag | <10 ppm |
| | nach 2 Monaten | <10 ppm |

Auch die Beispiele 6 bis 8 zeigen bei langer Versuchsdauer (2 Monate) eine erhebliche Veringerung der Migrationsneigung.

## Patentansprüche

1. Bicyclische Amidine der allgemeinen Formel 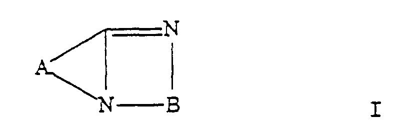
worin A ausgewählt ist aus der Gruppe -CR¹R²-CR³R⁴-CR⁵R⁶-, -CR¹R²-CR³R⁴-CR⁵R⁶-CR⁷R⁸-, -CR¹R²-CR³R⁴-CR⁵R⁶-CR⁷R⁸-CR⁹R¹⁰-, -worin die Substituenten in A jeweils vom Stickstoffatom ausgehend numeriert sind
und B ausgewählt ist aus der Gruppe -CR¹¹R¹²-CR¹³R¹⁴-, -CR¹¹R¹²-CR¹⁵R¹⁶-CR¹³R¹⁴-, -CR¹¹R¹²-CR¹⁵R¹⁶-CR¹⁷R¹⁸-CR¹³R¹⁴-
und R¹, R² und R¹¹ bis R¹⁴ jeweils unabhängig voneinander Wasserstoff, C₁-C₄-Alkyl, Phenyl, alkylsubstituiertes Phenyl oder mit Hydroxy, Amino, C₁-C₄-Alkylamino oder Mercapto substituiertes C₁-C₄-Alkyl und R³ bis R¹⁰ und R¹⁵ bis R¹⁸ jeweils unabhängig voneinander Wasserstoff, C₁-C₄-Alkyl, Phenyl, alkylsubstituiertes Phenyl, Hydroxy, Amino, C₁-C₄-Alkylamino, Mercapto oder mit Hydroxy, Amino, C₁-C₄-Alkylamino oder Mercapto substituiertes C₁-C₄-Alkyl bedeuten, mit der Maßgabe, daß wenigstens einer der Substituenten R¹ bis R¹⁸ Hydroxy, Amino, C₁-C₄-Alkylamino, Mercapto und/oder mit Hydroxy, Amino, C₁-C₄-Alkylamino oder Mercapto substituiertes C₁-C₄-Alkyl ist,
sowie deren Gemische, ausgenommen die Verbindung 7-Hydroxy-2-phenyl-2,3,5,6,7,8-hexahydroimidazo[1,2-a] pyridin.

2. Bicyclische Amidine nach Anspruch 1, **dadurch gekennzeichnet, daß** wenigstens einer der Substituenten R¹¹ bis R¹⁸ Amino oder Aminomethyl ist.

3. Bicyclisches Amidin nach Anspruch 1, **dadurch gekennzeichnet, daß** es 3-Amino-2,3,4,6,7,8-hexahydropyrrolo[1,2-a]pyrimidin der Formel 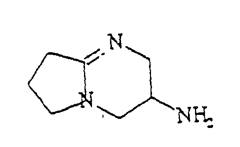 ist.

4. Bicyclisches Amidin nach Anspruch 1, **dadurch gekennzeichnet, daß** es 3-(Aminomethyl)-2,5,6,7-tetrahydro-3*H*-pyrrolo[1,2-a]imidazol der Formel 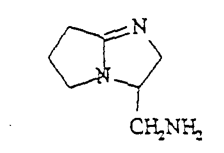 ist.

5. Bicyclisches Amidin nach Anspruch 1, **dadurch gekennzeichnet, daß** es 3-(Aminomethyl)-3-methyl-2,3,4,6,7,8-; hexahydropyrrolo[1,2-a]pyrimidin der Formel 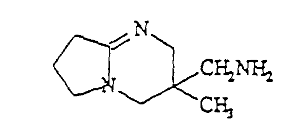 ist.

6. Verwendung der bicyclischen Amidine gemäß Ansprüchen 1 bis 5 als Katalysatoren bei der Herstellung von Polyurethanen.

7. Verfahren zur Herstellung bicyclischer Amidine der allgemeinen Formel 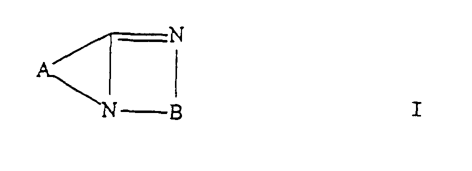 worin A und B die in Anspruch 1 genannte Bedeutung haben, **dadurch gekennzeichnet, daß** ein Lacton der allgemeinen Formel 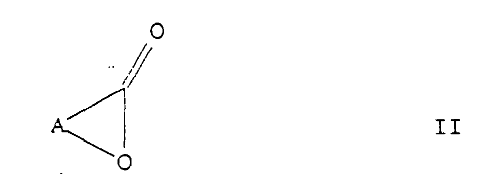 worin A die oben genannte Bedeutung hat, mit einer mindestens äquimolaren Menge eines Amins der allgemeinen Formel
H₂N―B―NH₂ III
worin B die oben genannte Bedeutung hat, auf wenigstens 150°C erhitzt und das so erhaltene Reaktionsgemisch ohne Isolierung eines Zwischenprodukts einer fraktionierten Destillation unterworfen wird.

8. Verfahren nach Anspruch 7, **dadurch gekennzeichnet, daß** die Umsetzung des Lactons (II) mit dem Amin (III) in Gegenwart eines sauren Katalysators durchgeführt wird.

9. Verfahren nach Anspruch 7 oder 8, **dadurch gekennzeichnet, daß** das Amin (III) in einer Menge von 2 bis 20 mol auf 1 mol des Lactons (II) eingesetzt wird.

10. Verfahren nach einem oder mehreren der Ansprüche 7 bis 9, **dadurch gekennzeichnet, daß** als Lacton (II) eine Verbindung aus der Gruppe γ-Butyrolacton, γ-Valerolacton, δ-Valerolacton, ε-Caprolacton, Pantolacton eingesetzt wird.

11. Verfahren nach einem oder mehreren der Ansprüche 7 bis 10, **dadurch gekennzeichnet, daß** als Amin (III) eine Verbindung aus der Gruppe 1,2-Diaminoethan, 1,2-Diaminopropan, 1,3-Diaminopropan, 1,2,3-Triaminopropan, 1,1,1-Tris(aminomethyl)ethan, Tetrakis(aminomethyl)methan eingesetzt wird.

## Claims

1. Bicyclic amidines of the general formula 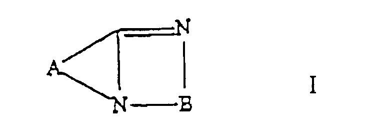
wherein A is selected from the group
-CR¹R²-CR³R⁴-CR⁵R⁶-, -CR¹R²-CR³R⁴-CR⁵R⁶-CR⁷R⁸-,
-CR¹R²-CR³R⁴-CR⁵R⁶-CR⁷R⁸-CR⁹R¹⁰-, wherein the substituents in A are each numbered starting from the nitrogen atom
and B is selected from the group
-CR¹¹R¹²-CR¹³R¹⁴, -CR¹¹R¹²-CR¹⁵R¹⁶-CR¹³R¹⁴-,
-CR¹¹R¹²-CR¹⁵R¹⁶-CR¹⁷R¹⁸-CR¹³R¹⁴-
and R¹, R² and R¹¹ to R¹⁴, each independently of one another, signify hydrogen, C₁-C₄ alkyl, phenyl, alkyl-substituted phenyl or C₁-C₄ alkyl substituted with hydroxy, amino, C₁-C₄ alkylamino or mercapto and R³ to R¹⁰ and R¹⁵ to R¹⁸, each independently of one another, signify hydrogen, C₁-C₄ alkyl, phenyl, alkyl-substituted phenyl, hydroxy, amino, C₁-C₄ alkylamino, mercapto or C₁-C₄ alkyl substituted with hydroxy, amino, C₁-C₄ alkylamino or mercapto, with the proviso that at least one of the substituents R¹ to R¹⁸ is hydroxy, amino, C₁-C₄ alkylamino, mercapto and/or C₁-C₄ alkyl substituted with hydroxy, amino, C₁-C₄ alkylamino or mercapto,
and mixtures thereof, except the compound 7-hydroxy-2-phenyl-2,3,5,6,7,8-hexahydroimidazo[1,2-a]pyridine.

2. Bicyclic amidines according to claim 1, **characterised in that** at least one of the substituents R¹¹ to R¹⁸ is amino or aminomethyl.

3. Bicyclic amidine according to claim 1, **characterised in that** it is 3-amino-2,3,4,6,7,8-hexahydropyrrolo[1,2-a]pyrimidine of the formula

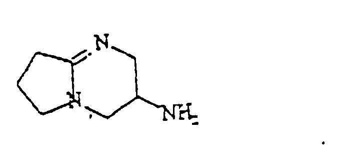

4. Bicyclic amidine according to claim 1, **characterised in that** it is 3-(aminomethyl)-2,5,6,7-tetrahydro-3*H*-pyrrolo[1,2-a]imidazole of the formula

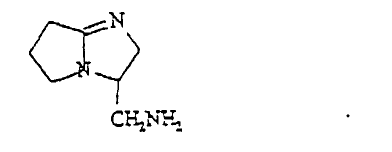

5. Bicyclic amidine according to claim 1, **characterised in that** it is 3-(aminomethyl)-3-methyl-2,3,4,6,7,8-hexahydropyrrolo[1,2-a]pyrimidine of the formula

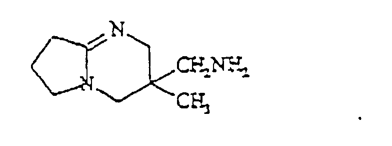

6. Use of the bicyclic amidines according to claims 1 to 5 as catalysts in the production of polyurethanes.

7. Process for the production of bicyclic amidines of the general formula 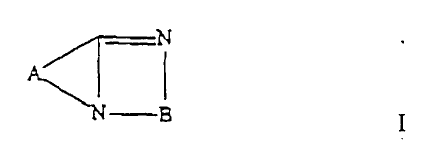 wherein A and B have the meaning given in claim 1, **characterised in that** a lactone of the general formula 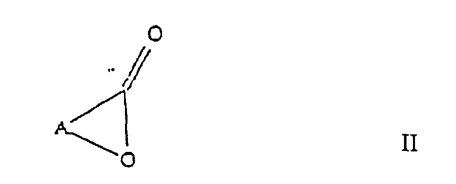 wherein A has the above meaning, is heated with an at least equimolar quantity of an amine of the general formula
H₂N―B―NH₂ III
wherein B has the above meaning, to at least 150°C and the reaction mixture thus obtained is subjected to a fractional distillation without isolating an intermediate product.

8. Process according to claim 7, **characterised in that** the reaction of the lactone (II) with the amine (III) is performed in the presence of an acidic catalyst.

9. Process according to claim 7 or 8, **characterised in that** the amine (III) is used in a quantity of 2 to 20 moles per 1 mole of the lactone (II).

10. Process according to one or more of claims 7 to 9, **characterised in that** a compound from the group γ-butyrolactone, γ-valerolactone, δ-valerolactone, ε-caprolactone, pantolactone, is used as the lactone (II).

11. Process according to one or more of claims 7 to 10, **characterised in that** a compound from the group 1,2-diaminomethane, 1,2-diaminopropane, 1,3-diaminopropane, 1,2,3-triaminopropane, 1,1,1-tris(aminomethyl)ethane, tetrakis-(aminomethyl)methane, is used as the amine (III).

## Revendications

1. Amidines bicycliques de formule générale 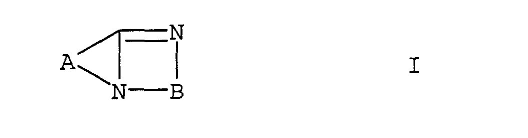
où A est choisi dans le groupe
-CR¹R²-CR³R⁴-CR⁵R⁶-, -CR¹R²-CR³R⁴-CR⁵R⁶-CR⁷R⁸-,
-CR¹R²-CR³R⁴-CR⁵R⁶-CR⁷R⁸-CR⁹R¹⁰-, où les substituants dans A sont numérotés à chaque fois à partir de l'atome d'azote
et B est choisi dans le groupe
-CR¹¹R¹²-CR¹³R¹⁴-, -CR¹¹R¹²-CR¹⁵R¹⁶-CR¹³R¹⁴-
CR¹¹R¹²-CR¹⁵R¹⁶-CR¹⁷R¹⁸-CR¹³R¹⁴-
et R¹, R² et R¹¹ à R¹⁴ représentent chacun indépendamment des autres l'hydrogène, alkyle en C₁-C₄, phényle, phényle alkyl-substitué ou alkyle en C₁-C₄ substitué par hydroxyle, amino, alkyle en C₁-C₄-amino ou mercapto et R³ à R¹⁰ et R¹⁵ à R¹⁸ représentent chacun indépendamment des autres l'hydrogène, alkyle en C₁-C₄, phényle, phényle alkyl-substitué, hydroxyle, amino, alkyle en C₁-C₄-amino, mercapto ou alkyle en C₁-C₄ substitué par hydroxyle, amino, alkyle en C₁-C₄-amino ou mercapto, à condition qu'au moins l'un des substituants R¹ à R¹⁸ soit hydroxyle, amino, alkyle en C₁-C₄-amino, mercapto et/ou alkyle en C₁-C₄ substitué par hydroxyle, amino, alkyle en C₁-C₄-amino ou mercapto,
ainsi que leurs mélanges, à l'exception du composé 7-hydroxy-2-phényl-2,3,5,6,7,8-hexahydroimidazo[1,2-a]-pyridine.

2. Amidines bicycliques selon la revendication 1 **caractérisées en ce qu'**au moins l'un des substituants R¹¹ à R¹⁸ est amino ou aminométhyle.

3. Amidine bicyclique selon la revendication 1 **caractérisée en ce qu'**il s'agit de la 3-amino-2,3,4,6,7,8-hexahydropyrrolo[1,2-a]pyrimidine de formule

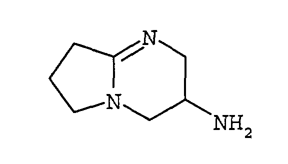

4. Amidine bicyclique selon la revendication 1 **caractérisée en ce qu'**il s'agit du 3-(aminométhyl)-2,5,6,7-tétrahydro-3H-pyrrolo[1,2-a]-imidazole de formule

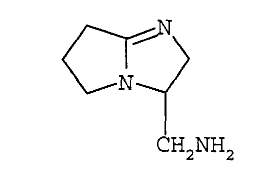

5. Amidine bicyclique selon la revendication 1 **caractérisée en ce qu'**il s'agit de la 3-(aminométhyl)-3-méthyl-2,3,4,6,7,8-hexahydropyrrolo-[1,2-a]pyrimidine de formule

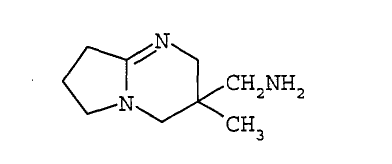

6. Utilisation des amidines bicycliques selon les revendications 1 à 5 comme catalyseurs dans la préparation de polyuréthanes.

7. Procédé de préparation d'amidines bicycliques de formule générale 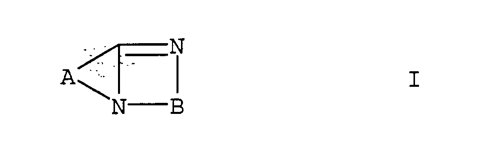 où A et B ont la signification citée dans la revendication 1, **caractérisé en ce qu'**une lactone de formule générale 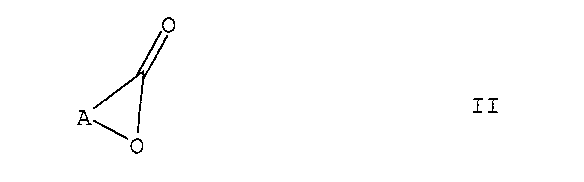 où A a la signification citée ci-dessus est chauffée à au moins 150°C avec une quantité au moins équimolaire d'une amine de formule générale
H₂N-B-NH₂ III
où B a la signification citée ci-dessus, et le mélange réactionnel ainsi obtenu est soumis à une distillation fractionnée sans isolement d'un produit intermédiaire.

8. Procédé selon la revendication 7 **caractérisé en ce que** la réaction de la lactone (II) avec l'amine (III) est conduite en présence d'un catalyseur acide.

9. Procédé selon la revendication 7 ou 8 **caractérisé en ce que** l'amine (III) est utilisée en une quantité de 2 à 20 moles par mole de lactone (II).

10. Procédé selon une ou plusieurs des revendications 7 à 9 **caractérisé en ce que** l'on utilise comme lactone (II) un composé du groupe de la γ-butyrolactone, de la γ-valérolactone, de la δ-valérolactone, de l'ε-caprolactone, de la pantolactone.

11. Procédé selon une ou plusieurs des revendications 7 à 10 **caractérisé en ce que** l'on utilise comme amine (III) un composé du groupe du 1,2-diaminoéthane, du 1,2-diaminopropane, du 1,3-diaminopropane, du 1,2,3-triaminopropane, du 1,1,1-tris(aminométhyl)éthane, du tétrakis(aminométhyl)-méthane.
